(19) Europäisches Patentamt
European Patent Office
Office européen des brevets

(11) Publication number: 0 025 569
B1

(12) **EUROPEAN PATENT SPECIFICATION**

(45) Date of publication of patent specification: **20.03.85**

(51) Int. Cl.[4]: **C 07 F 7/08**, C 07 F 1/00, C 07 F 3/02, A 61 K 31/695

(21) Application number: **80105311.7**

(22) Date of filing: **05.09.80**

(54) **Silicon-bearing amides, their production, intermediates in their production, and pharmaceutical compositions containing them.**

(30) Priority: **10.09.79 US 74002**
**15.04.80 US 140562**
**24.04.80 US 143262**

(43) Date of publication of application:
**25.03.81 Bulletin 81/12**

(45) Publication of the grant of the patent:
**20.03.85 Bulletin 85/12**

(84) Designated Contracting States:
**BE CH DE FR GB IT LI NL SE**

(56) References cited:
**EP-A-0 012 183**
**EP-A-0 013 818**
**FR-A-1 019 750**
**FR-A-1 033 063**
**US-A-2 973 383**

**CHEMICAL ABSTRACTS, vol. 71, no. 25, December 22, 1969, pages 480-481, abstract 124566z Columbus, Ohio, US R.I. PAL'CHIK et al.: "Silicon containing alkxyacetylenes"**
**CHEMICAL ABSTRACTS, vol. 63, no. 13, December 20, 1965, abstract 18138g Columbus, Ohio, US L.L. SHCHUKOVSKAYA et al.: "Synthesis and reactions of trimethylsilkylketene: trimethylsiloxy-acetylene"**

(73) Proprietor: **SANDOZ AG**
**Lichtstrasse 35**
**CH-4002 Basel (CH)**

(72) Inventor: **Damon, Robert Edson II**
**44 Centergrove Road**
**Randolph, N.J. 07869 (US)**
Inventor: **Barcza, Sandor**
**8 Larchdell Way**
**Mountain Lakes, N.J. 07046 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 025 569 B1**

**Description**

This application relates to novel silicon-bearing amides, processes for their production and their use as pharmaceuticals.

In particular the invention relates to compounds of formula I

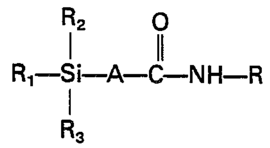

I

wherein R represents

a) an aralkyl radical of formula

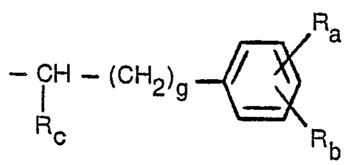

in which

g represents 0, 1 or 2,

$R_a$ represents hydrogen, fluorine, chlorine, bromine or iodine, $(C_{1-4})$alkyl or alkoxy or trifluoromethyl,

$R_b$ represents hydrogen, fluorine or chlorine, or $(C_{1-3})$alkyl or alkoxy,

$R_c$ represents

i) hydrogen

ii) a radical of formula

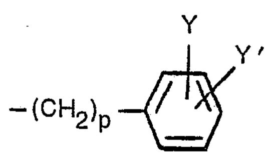

in which p represents 0, 1 or 2, and

Y represents hydrogen, fluorine, chlorine, bromine or iodine, or $(C_{1-4})$alkyl or alkoxy,

Y' represents hydrogen, fluorine or chlorine or $(C_{1-3})$ alkyl or alkoxy, or

iii) $(C_{1-8})$alkyl,

or R represents

b) an indolyl radical of formula

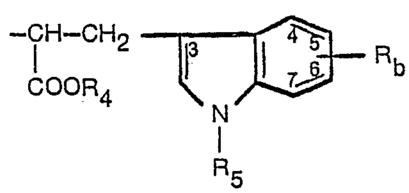

in which

$R_b$ is as defined under a)

$R_4$ represents $(C_{1-8})$alkyl or unsubstituted benzyl, and

$R_5$ represents hydrogen, $(C_{1-8})$alkyl or unsubstituted benzyl,

or R represents

c) a benzocycloalkyl radical of formula

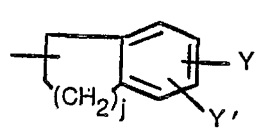

in which

Y and Y′ are as defined under a) and

j represents 1, 2, 3 or 4,

$R_1$, $R_2$ and $R_3$ represent independently $C_{1-22}$alkyl or a radical of the formula

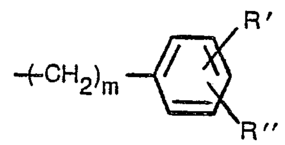

in which m represents 0, 1 or 2 and

R′ and R″ represent independently hydrogen, $C_{1-3}$alkyl or alkoxy, fluorine, chlorine, bromine or iodine or

$R_1$ is as defined above and $R_2$ and $R_3$ together with the silicon atom to which they are attached form a 3 to 20 membered ring, and A is a $C_{2-20}$-alkylene group.

The compounds for formula I can be prepared by acylating a compound of formula III

$H_2NR$ III

with a compound of formula IV

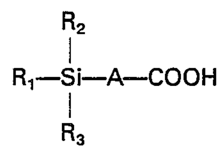

IV

or a reactive derivative thereof such as an active ester, carboimide adduct or a mixed anhydride of formula II

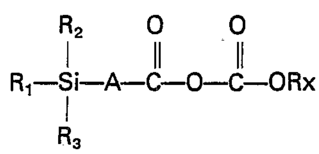

II

15 or an acid halide of formula II′

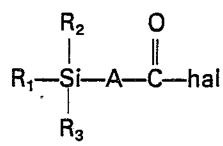

II′

The reaction of III with IV or II and the preparation IV can be carried out analogously to the method described in EP—A—0 012 183.

The compounds of formula II′ can be prepared in conventional manner by halogenating a compound of formula IV e.g. with thionyl chloride and optionally in the presence of dimethylformamide as catalyst.

Reaction of III with II′ is carried out in an acid binding agent such as pyridine, triethylamine, diisopropylamine preferably triethylamine and at temperatures of e.g. 10 to 50°C, especially 20 to 30°C. In the formulae II, II′, III and IV $R_1$, $R_2$, $R_3$, R and A are as defined above, $R_x$ represents $C_{1-4}$alkyl e.g. methyl or ethyl and hal represents chlorine or bromine.

Compounds of formula IV wherein $R_1$, $R_2$ and $R_3$ are $C_{1-22}$alkyl and A is ethylene are disclosed in EP—A—0 012 183 and those wherein at least one of $r_1$, $R_2$, $R_3$ is phenyl and the others(s) methyl and A is ethylene is disclosed in JACS *76*, 1609—12. The remaining compounds of formula IV are new. Alternative methods for preparing the compounds of formula IV are illustrated by the following reaction schemes D and D′.

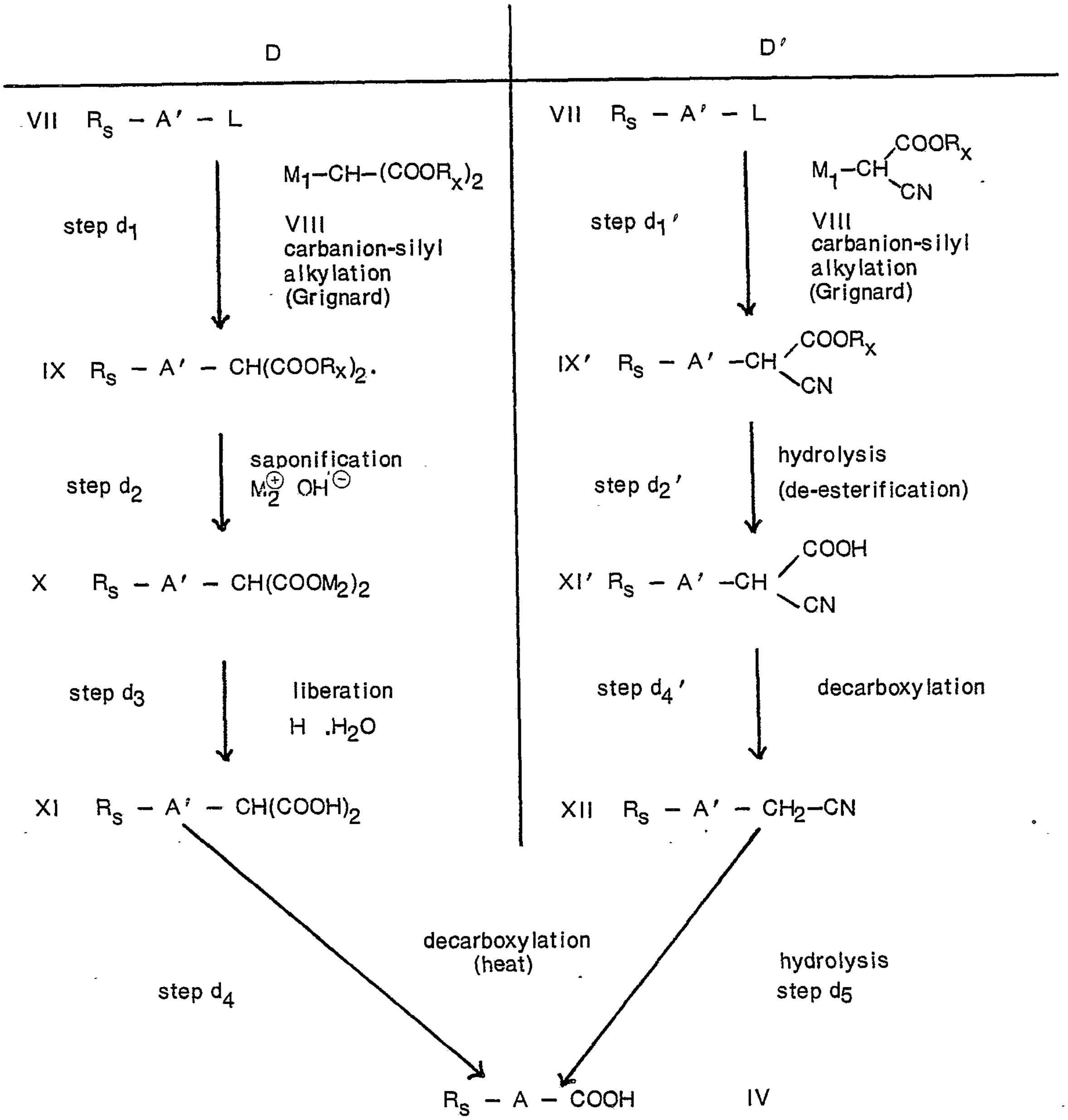


whereby

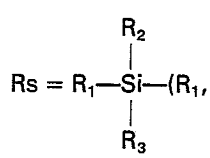 (R₁, R₂, R₃ as defined above)

A' = a group corresponding to A but having one less carbon atom
$R_x$ = as defined above
L = leaving group e.g. Cl, Br, I or a sulfonate e.g. methanesulfonate, p-toluenesulfonate
$M_1$ = equivalent of an active metal e.g. Li, K, Na or MgBr or MgCl
$M_2$ = equivalent of a cation of a strong base e.g. potassium.

The processes outlined above can be carried out analogously to conventional processes of this type e.g. as illustrated in the examples. Suitable conditions are e.g. in all cases solvents which are inert under reaction conditions and for

$d_1$, $d_1'$ : 10° to 80°C especially 45—65°C' anhydrous medium e.g. dimethylformamide or poly- or cyclic ethers e.g. tetrahydrofuran

$d_4$, $d_4'$ : 60° to 200°C and conveniently reduced pressure

$d_2$, $d_3$ : conventional manner

$d_2'$ : excess acetic acid
$d_5$ : two stages ① hydrolysis of XII to a salt ② neutralization.

It will be appreciated that where possible reagents will be prepared in situ and reaction steps condensed. Thus, e.g. preparation of VIII and VII, their reaction to IX and decarboxylation of IX may all be carried out in the same vessel.

An alternative method for preparing compounds of formula IV in which A contains 2 to 9 carbon atoms is illustrated below.

REACTION SCHEME E

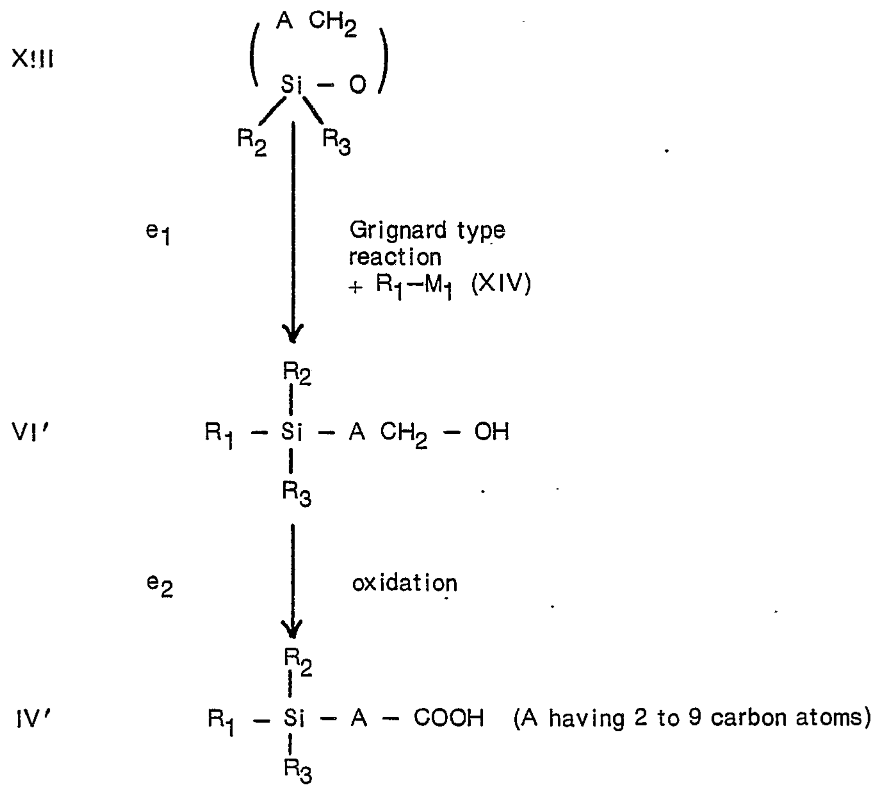


whereby $R_1$, $R_2$, $R_3$, A and $M_1$ are as defined above.

Conventional conditions and inert media are employed e.g. as illustrated in the examples. Suitable conditions for the individual steps are e.g.

$e_1$ : 10° to 100°C especially 20°C to reflux
$e_2$ : −40° to +30° especially −10° to +5°C; acetone with water as solvent; $KM_nO_4$ or $CrO_3$ in acidic conditions e.g. phosphoric, acetic or especially sulfuric acid as oxidation catalyst.

Additional processes for preparing compounds of formula I are illustrated in the following reaction schemes F and G wherein $R_1$, $R_2$, $R_3$, R, A, $R_s$, $R_x$, $M_1$ are as defined above, Z stands for fluoro, chloro, bromo or iodo and A''' represents a group corresponding to A but having two carbon atoms less.

It will be appreciated that in the various hydrogenation processes conditions may be chosen whereby the alkynyl compounds of formulae XVI, XXII may either be reacted directly to compounds of formulae I and XXIII (total hydrogenation) or only partially hydrogenated to compounds of formulae XV and XXIV (controlled hydrogenation).

REACTION SCHEME F

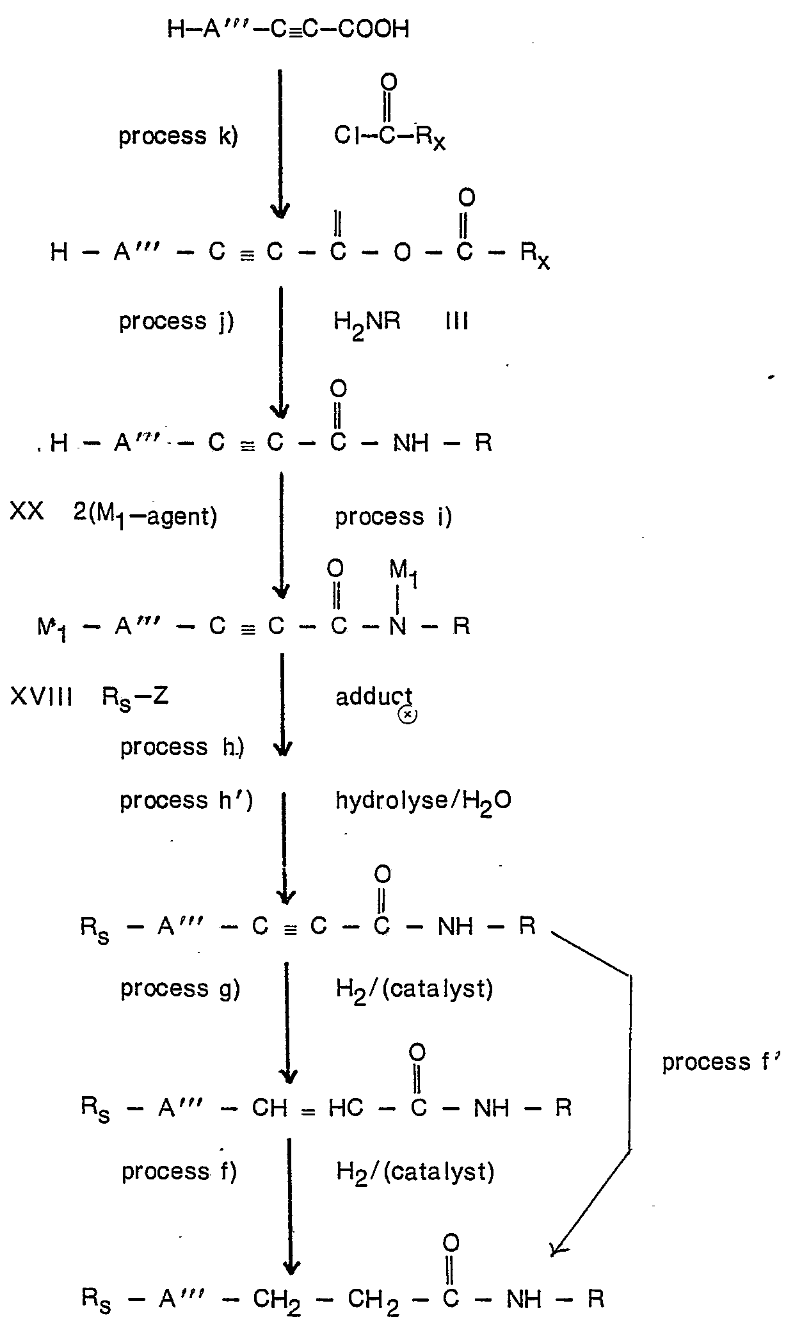


XXI

XIX

XVII

XVI

XV

I

REACTION SCHEME G

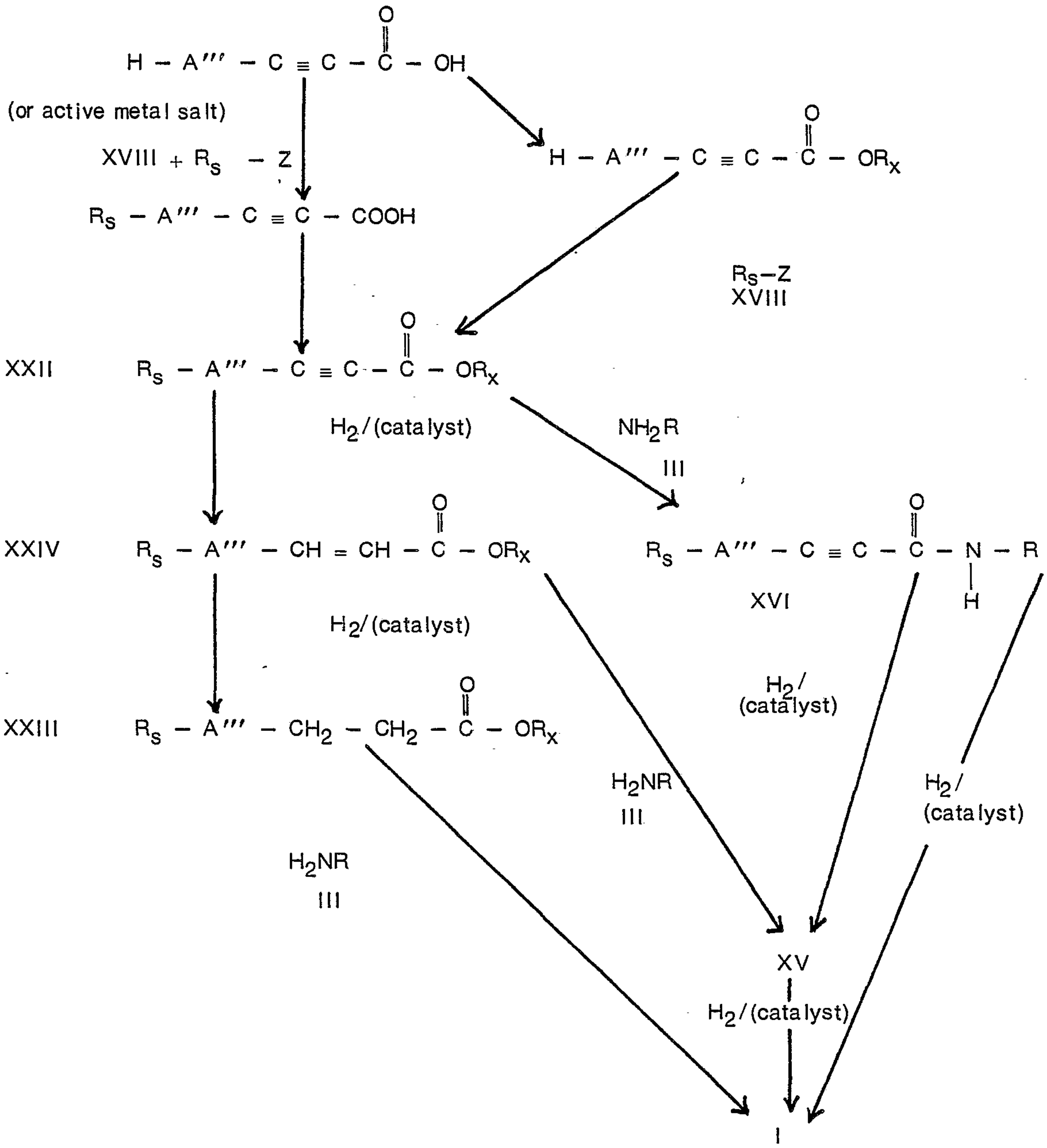


It will be appreciated that the conditions employed in the above outlined procedures F are similar to those conventionally employed for known reactions of the same type. The reactions are usually carried out in solvents inert under the reaction conditions. Examples of suitable conditions are given in the following table and in the examples.

| Process | temperature | solvents | others |
|---|---|---|---|
| g)<br>(hydrogenation) | 10° to 80°C<br>particularly<br>20° to 30°C | lower alcohols e.g. ethanol; lower fatty acids + esters e.g. acetic acid, ethylacetate; hydrocarbons e.g. benzene toluene or cyclic ethers e.g. tetrahydrofuran | pressure atmospheric 7 bar over atmospheric<br>$H_2$ + catalyst such as $Pd/CaCO_3$ or Rh, Pt or PtO on $CaCO_3$, $BaSO_4$ or charcoal |
| f and f′<br>(hydrogenation) | 10° to 100°C<br>particularly<br>20 to 30°C | as for a) | pressure 1 to 7 bar, particularly 3.5 bar over atmospheric<br>catalysts as for g) |
| h)<br>(Grignard) | moderate<br>e.g. −40°—0°C | anhydrous aprotic: ethers e.g. tetrahydrofuran, dimethoxyethane; hydrocarbons e.g. benzene, toluene, hexane | inert gas such as dry nitrogen |
| h′)<br>hydrolysis | 5—90°C<br>particularly<br>20 to 30°C | conventional | for hydrolysis e.g. water, dilute aqueous acid or salt (e.g. $NH_4Cl$) |
| i<br>introduction of $M_1$ | reduced e.g. −78° to −20°C particularly −60°C | as h) | compound XVII can be used directly without isolation in process h). suitable agent for introducing $M_1$ is e.g. lithium diisopropylamide prepared in situ in conventional manner |
| j) | → | → | as described above for reaction of compounds of formula III with those of formulae IV, II and II′ as described above for preparing II. |
| k) | → | → | |

The conditions for the process G are similar to those described above for analogous processes and in the examples.

The novel intermediates of the formulae XV and XVI and their hydrogenation to compounds of the formula I form part of the invention. The preferred compounds of these types are equivalent to those hereinafter described for formulae I and Ia. The intermediates of formulae XVII, VI′, IX and IX′ are also novel.

The remaining intermediates are either known or can be prepared in conventional manner from commercially available starting materials.

The compounds obtained in the various steps of the processes may be isolated and purified using conventional techniques.

It will be understood that the compounds of formula I and their precursors may exist in the form of optically active isomers, e.g., enantiomers, which can be prepared from optically active starting compounds or separated and recovered by conventional techniques, i.e., resolution. Such isomeric forms are also included within the scope of this invention and unless otherwise stated compounds are named as racemates.

The compounds of the formula I wherein $R_1$, $R_2$ and $R_3$ each represent $C_{1-22}$alkyl and A represents an ethylene radical are covered by EP—A—0 012 183. The remaining compounds of formula I are new and also form part of the invention.

The invention therefore provides compounds of formula Ia

$$R_1-\underset{R_3}{\overset{R_2}{Si}}-B-\overset{O}{\overset{\|}{C}}-NH-R \qquad \text{Ia}$$

wherein $R_1$, $R_2$, $R_3$ and R are as defined above and B represents a $C_{2-20}$alkylene radical whereby when B represents ethylene at least one of $R_1$, $R_2$, $R_3$ is other than $C_{1-22}$alkyl.

The compounds of formula Ia and XV possess pharmacological activity. In particular they are indicated for use in controlling the cholesterol ester content of mammalian arterial walls and are therefore particularly indicated for use as anti-atherosclerotic agents, i.e. agents useful in the prophylactic treatment of atherosclerosis and in the controlling of atherosclerotic conditions due to cholesterol ester accumulation in the arterial walls. Such ability of the compounds of the formula I is indicated by oral administration (gavage) at a dose of 200 mg/kg of the test compound per day for 9 weeks to rabbits in conjunction with a high cholesterol (e.g. 2%) diet resulting in, compared to controls, a reduction in cholesterol and cholesterol ester levels particularly a lessened formation or absence of arterial wall plaques. The serum cholesterol and cholesterol ester levels are determined by conventional methods, e.g. serum samples are collected, and 1.0 ml samples of the serum are added to 9.0 ml of redistilled isopropanol; an aliquot is taken for analysis by the method described by Heider and Boyett in J. of Lipid Research, *19,* 514 (1978).

The said ability of the compounds is also shown in test procedures in which the total cholesterol ester content of cultured cells is shown to be reduced by a test compound.

The invention therefore also concerns compounds of formula Ia (or XV) for use as pharmaceuticals e.g. as anti-atherosclerotic agents.

An indicated suitable daily dosage is from about 10 to 5,000 mg preferably 10 to 2,000 mg suitably administered in divided doses of 2.5 to 2,500 mg, two to four times daily, or in retard form.

The compounds of formula Ia or XV may be administered alone, or in admixture with a pharmaceutically acceptable diluent or carrier, and, optionally other excipients, and administered orally in such forms as tablets, elixirs, capsules or suspensions or parenterally in such forms as injectable solutions or suspensions.

The preferred pharmaceutical compositions from the stand-point of ease of preparation and administration are solid compositions, particularly tablets and hard-filled or liquid-filled capsules.

Such compositions also form part of the invention.

Particular embodiments of the invention are compounds of the formula I wherein

(i) R, is as defined above, $R_1$, $R_2$ and $R_3$ represent independently a) $C_{1-22}$alkyl or b) a radical of the formula

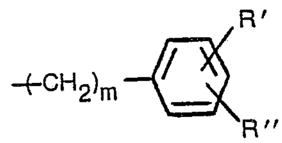

as defined above and A represents an ethylene group, whereby at least one of $R_1$, $R_2$ and $R_3$ is other than alkyl, or

(ii) R is as defined above,

$R_1$, $R_2$ and $R_3$ represent independently a) $C_{1-22}$alkyl or b) a radical of formula

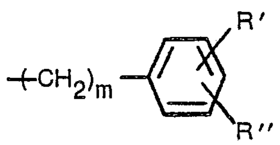

as defined above and A represents a $C_{3-20}$alkylene radical, whereby the sum of the carbon atoms in A and any one of $R_1$, $R_2$ and $R_3$ is not greater than 34, or

(iii) R and $R_1$ are as defined above, $R_2$ and $R_3$ together with the silicon which they are attached form a 3 to 20 membered ring and A represent an ethylene radical.

Within groups (i) and (ii) above, compounds are preferred wherein two of $R_1$, $R_2$, $R_3$ are the same and not more than two are of type b), e.g. compounds wherein $R_1=R_2$ = type b) wherein m = 0 and $R_3$ = type a) such as n-butyl and n-decyl.

Within the group (ii) above, compounds are in addition preferred wherein $R_1$, $R_2$ and $R_3$ are independently $C_{1-22}$alkyl e.g. wherein $R_1$ is unbranched alkyl of 8 to 18 carbon atoms such as n-decyl and $R_2$ and $R_3$ are each $C_{1-3}$alkyl e.g. methyl.

Within the preferred groups of compounds (ii), A contains preferably 3 to 10 especially 3 to 6 carbon atoms.

Within the group (iii) above, compounds are preferred wherein $R_1$ is unbranched alkyl of 8 to 18 carbon atoms such as n-decyl and $R_2$ and $R_3$ together especially form e.g. a saturated optionally branched hydrocarbon radical preferably having no more than 25 carbon atoms such as an optionally methyl or ethyl substituted polymethylene chain having 2 to 19, preferably 2 to 13, particularly 2 to 7, especially 4 or 5 carbon atoms in the chain. Unsubstituted chains are preferred.

The total sum of carbon atoms in $R_1$, $R_2$ and $R_3$ is preferably not more than 35.

Within all the above mentioned particular embodiments and preferred groups of the invention, the following meanings of R are preferred.

R is preferably of type a).

When R is of type a), where g = 1 and $R_c$ is of type ii) where p = 0, then R can be an 1-(phenyl)-2-(*p*-tolyl)ethyl radical and when $R_c$ is of type ii) where p = 1, then R can be a 1-(benzyl)-2-phenylethyl radical.

When R is of type b), it is preferred that, when the phenyl ring is mono-substituted, the substitutent be located at the 5-position of the indole nucleus. It is also preferred that when $R_4$ is alkyl, it is unbranched, particularly ethyl.

When R is of type c), it is preferred that when Y is other than a hydrogen atom, that it be located at a carbon atom ortho to the ring junction and that when Y' is also other than a hydrogen, it is preferred that it be the same as Y, and it is additionally preferred that it be in para-position to Y.

It is additionally preferred that the amide group be linked to a carbon of the cycloalkyl moiety which is directly bonded to a ring junction carbon. It is also preferred that j be 1, i.e. that the benzocycloalkyl nucleus be indanyl and particularly 1-indanyl.

In the above-presented definitions, when $R_a$ or Y is halo, it is preferably fluorine or chlorine and particularly chlorine, and when $R_2$ or Y' is florine or chlorine, it is preferably chlorine.

The above preferences and combinations of them also form part of the invention.

The examples illustrate the invention, temperatures being in degrees centigrade.

Example 1

4,4-Diphenyl-4-sila-N-(1'-phenyl-2'-p-tolyl)-ethyl)valeramide (compound No. 1).

*Step A* N-[(1'-phenyl-2'-p-tolyl)-ethyl]-propiolamide (compound XIXa)

95 mg of lithium hydride are added to a solution of 850 mg propiolic acid in 15 ml of freshly distilled THF, portion-wise, over a period of about 45 min., with cooling to avoiding heating over room temperature. The resulting mixture is then cooled to about −10°, and a solution of 1.3 g of ethyl chloroformate in 3 ml of dry THF is added drop-wise with stirring, while maintaining the temperature below −10°. The resulting mixture is then stirred for 2 hrs. at about −15°. A solution of 2.5 g of (1-phenyl-2-p-tolyl)ethylamine in 5 ml of dry THF is then added dropwise at from −15° to 0°, with stirring. The mixture is then stirred at room temperature for 2 hours.

The reaction mixture is concentrated by evaporation in vacuo to obtain a residue, which is taken up in methylene chloride and is washed first with dilute aqueous sodium bicarbonate, then with dilute hydrochloric acid, then dried over anh. sodium sulfate, and concentrated in vacuo to obtain crude product. The crude product of this step is refined by crystallizing from diethyl ether m.p. 152—155°.

*Step B* 4,4-Diphenyl-4-sila-N-(1'-phenyl-2'-p-tolylethyl)-2-pentynamide (compound XVIa)

In a vessel, under an atmosphere of dry nitrogen at −30° 460 mg of n-butyl lithium in 10 ml of dry hexane is added to 300 mg of diisopropylamine in 15 ml of dry THF. The mixture stirred for 15 min., then cooled to −60°. A solution of 600 mg of N-(1'-phenyl-2'-p-tolyl)-ethyl] propiolamide in 10 ml of dry THF is added dropwise thereto while the temperature of the mixture is maintained below −50°. The mixture is then stirred for 1 hr. at −20°. A solution of 550 mg of diphenyl-methylchlorosilane in 3 ml of dry THF is added dropwise, with stirring and the mixture stirred for 90 min (at −20°).

Aqueous saturated ammonium chloride is added to the reaction mixture, and the organic phase recovered, dried over anh. sodium sulfate, and concentrated by evaporation in vacuo to obtain the crude product as an oil, which is refined by eluting through a silica gel column using chloroform as eluate to yield the product of this step as an oil.

*Step C* 4,4-diphenyl-4-sila-N-(1'-phenyl-2'-p-tolyl-ethyl)valeramide (compound No. 1)

To a solution of 300 mg of 4,4-diphenyl-4-sila-N-(1'-phenyl-2'-p-tolylethyl)-2-pentynamide in 30 ml of ethyl acetate in a hydrogenating apparatus, is added 50 mg of platinum oxide, and a pressure of 50 p.s.i. hydrogen is maintained for 24 hours with shaking. The reaction mixture is then filtered, and the filtrate concentrated (by evaporation in vacuo) to obtain the title product as an oil.

Example 2

4,4-Dimethyl-4-sila-N-(1'-phenyl-2'-p-tolylethyl-2-pentenamide (compound XVa).

*Step A & B* 4,4-dimethyl-4-sila-N-(1'-phenyl-2'-p-tolylethyl)-2-pentynamide (compound XVIb)

Repeating the procedure of Steps A and B of Example 1, but using in place of the diphenylmethylchlorosilane used (in step B) therein, an approximately equivalent amount of trimethylchlorosilane, there is accordingly obtained 4,4-dimethyl-4-sila-N-(1'-phenyl-2'-p-tolylethyl)-2-pentynamide (m.p. 118—120°).

*Step C* 4,4-dimethyl-4-sila-N-(1'-phenyl-2'-p-tolylethyl)-2-pentenamide (compound XVa)

To a solution of 335 mg of 4,4-dimethyl-4-sila-N-(1'-phenyl-2'-p-tolylethyl)-2-pentynamide in 25 ml of ethanol in a hydrogenating apparatus, is added 50 mg of 5% palladium on calcium carbonate. The mixture is placed under 1 at. pressure of hydrogen and shaken until an equivalent of hydrogen gas had been taken up (about 45 min.). The reaction mixture is then filtered, and the filtrate concentrated (by evaporation in vacuo) to obtain the title product as an oil.

Example 3

6,6-Dimethyl-6-sila-N-(1'-benzyl-2'-phenylethyl)-hexadecanamide (compound No. 2).

*Step A* n-decyl-dimethyl-(3-chloropropyl)-silane (compound VIIa)

Under nitrogen 32.4 g of n-decyl bromide is added to 3.8 g of magnesium turnings, in 100 ml. of absolute tetrahydrofuran (THF) cautiously (over a period of about 0.5 hr.) with external cooling (cold water bath) as needed to maintain a temperature of below 38°. The resulting mixture is then refluxed for 10 min, (to produce a Grignard reagent) then cooled to 35°. The Grignard reaction mixture is then added to 27.3 g of (3-chloropropyl)-dimethyl-chlorosilane in 100 ml of absolute tetrahydrofuran, with temperatures maintained at below 15° (using a salt-ice bath). The mixture is then stirred at room temperature for 2 days then heated at near reflux for 1 h. The mixture is then cooled, poured into ice-water and the organic material extracted with methylene chloride. The extract is dried, and concentrated to obtain the crude product of this step as oily residue. The residue is distilled using a Vigreux column, to obtain refined product of this step as a main fraction recovered at 118°—125° at 0.2 mBar for use in the next step (B).

*Step B* n-Decyl-dimethyl-(3-iodopropyl)-silane (compound VIIb)

A mixture of 25.0 g of sodium iodide, 120 ml. of acetonitrile (dried over molecular sieves) and 26.4 g of n-decyl-dimethyl (3-chloropropyl)-silane is stirred at near the reflux temperature of the mixture (at about 80°) for 40 h. After cooling, the mixture is mixed with water and toluene, and the organic phase recovered, washed thrice with water, then dried and concentrated (to about 40 ml) of an oil which on distillation using a Kugelrohr apparatus yields as the main fraction, refined title product of this step at 83 to 139°, at 0.186—0.126 mBar (0.140—0.095 mm Hg) for use in the next step.

*Step C* Methyl 2-methoxycarbonyl-6,6-dimethyl-6-sila-n-hexadecanoate (compound IXa)

To a reaction vessel system filled with nitrogen, 964 mg of 61.1% sodium hydride in mineral oil is added, which is washed free of the oil by employing 3 portions of petroleum ether/hexane (1:1). The thus washed sodium hydride is then suspended in 20 ml of fresh hexane and added in several portions to 8.1 g of n-decyl-dimethyl-(3-iodopropyl)-silane plus 3.6 g of dimethyl malonate in 25 ml of dry dimethylformamide plus 25 ml of dry petroleum ether, with stirring and cooling at such a rate that hydrogen gas evolution is controlled, and the temperature is at 0° to 10° (new solids form). The reaction mixture is then stirred in a bath at 55° for 2 hrs. and then at 65° for 1 hr. After cooling to room temperature, 2 ml of acetic acid (85%) is added, followed by an excess of water and petroleum ether. The organic layer is separated, washed thrice with water, dried and then concentrated to obtain the crude title product of this step as an oily residue, which is distilled through a Vigreux column to obtain refined title product of this step at 135—139° at 0.14 mBar (0.105 mm Hg).

*Step D* 6,6-dimethyl-6-silahexadecanoic acid (compound IVa)

A mixture of 6.7 g of methyl 2-methoxycarbonyl-6,6-dimethyl-6-silahexadecanoate (from step C) in 50 ml of methanol and 3.0 g of potassium hydroxide (powder) in 50 ml of methanol is refluxed for 45 min. The resulting mixture is then concentrated to a solid. Dilute hydrochloric acid (2N) is then added to the solid (in excess, to acid pH) and the mixture extracted with methylene chloride. The extract is dried, then concentrated to a residue. The residue is heated to 160—170° under reduced pressure (approx. 15 mm Hg), for 1 hour, then distilled under high vacuum through a jacketed Vigreux column to obtain refined title product of this step (as the main fraction) at 145—140° at 0.134—0.12 mBar (0.101—0.090 mm Hg).

*Step E* 6,6-dimethyl-6-sila-N-(1′-benzyl-2′-phenylethyl)hexadecanamide (compound No. 2)

3.0 g of the hydrochloride salt of N-benzyl-2-phenylethylamine is distributed between dilute aqueous sodium hydroxide and methylene chloride. The organic phase (containing the free amine) is dried, then concentrated *in vacuo* and redissolved in 25 ml of dry methylene chloride and held for reaction with the acyl chloride, below.

0.025 ml of N,N-dimethylformamide is added to 1.6 g of 6,6-dimethyl-6-silahexadecanoic acid in 50 ml. of dry methylene chloride/toluene (1:1 v/v) and the resulting solution cooled (in an ice bath), and 1.5 ml of thionyl chloride added (with stirring). Stirring is continued for 0.5 h at 0° to 20°, then the mixture concentrated *in vacuo* to an oily residue (which is the acyl chloride), which is then dissolved in 25 ml of dry methylene chloride and cooled (with ice-water). The amine-containing solution is then mixed with the cold acyl chloride-containing solution. The resulting mixture (a slurry results) is stirred for 16 h at 20°, then the solvent removed by evaporation *in vacuo*, to obtain a residue, which is distributed between hexane and an excess of dilute hydrochloric acid. The organic phase is separated, then washed with two portions of dilute hydrochloric acid, then with three portions of dilute aq. sodium hydroxide, followed by one wash with sat. aq. sodium bicarbonate. The organic phase is then dried, and concentrated to an oily residue (crude product) which is then quick-chromatographed on silica gel, eluting with methylene chloride containing increasing concentrations of methanol. The title product is recovered as the main fraction (using 2% methanol) and obtained as a residue by heating in a rotating flask at 110° at 1.33 mBar (0.1 mm Hg). to remove solvent (eluant).

Example 4

5,5-dimethyl-5-silapentadecanoic acid (compound IVb)

*Step A* 5,5-dimethyl-5-silapentadecanol (compound VI′a)

A Grignard reagent is prepared by adding, under nitrogen, 50 g of n-decyl bromide to 6.5 g of magnesium turnings in 200 ml of absolute THF at 20—40°. After heating at 55°, 28.1 g of 1,1-dimethyl-1-sila-2-oxacyclohexane is added to the Grignard-reagent, and the mixture stirred for 20 h, at 60°. After cooling, the reaction mixture is poured into a mixture of saturated aq. ammonium chloride, ice and hexane. The organic phase is recovered and washed with water, then dried, and concentrated to the crude title product of this step (colorless), which is refined by distilling through a Vigreux column at 116—113°, at 2.53—2.33 mBar (0.190—0.175 mm Hg).

*Step B* 5,5-dimethyl-5-silapentadecanoic acid (compound IVb)

To a solution of 25.0 g of 5,5-dimethyl-5-silapentadecanol in 100 ml of pyridine, is added 21.5 g of potassium permanganate in 320 ml of pyridine, at 20—25° with stirring, and stirring is continued for 30 h. A dark brown precipitate forms which is filtered off (leaving a colorless filtrate). The filter cake is thoroughly

washed with methanol and ligroine. The washes and filtrate are combined and concentrated to near dryness. The residue is added to a mixture of water, ice, petroleum ether and dilute hydrochloric acid and the organic phase recovered. The aqueous phase is washed once with petroleum ether, which wash is then combined with the previously recovered organic phase, and the combined organic phases washed thrice with water, dried, and then concentrated to obtain crude title product, which is refined by distillation through a Vigreux column, b.p. 142—132° at 0.193—0.153 mBar (0.145—0.115 mm Hg).

Example 5

4-Sila-4,4-dimethyltetradecanoic Acid (compound IVc)

*Step A* ethyl 4-sila-2-cyano-4,4-dimethyltetradecanoate (compound IX'a)

Under anhydrous conditions, 1.161 g of sodium hydride dispersed in mineral oil (61.1% NaH) is washed free of the oil by washing thrice with portions of hexane. The thus washed sodium hydride is then added cautiously under a nitrogen atmosphere to a solution of 3.8 g of ethyl cyanoacetate and 9.6 g of n-decyl-dimethyl-iodomethylsilane in 20 ml of dry dimethyl formamide (which had been distilled from calcium hydride), with external cooling applied as necessary to avoid rapid temperature rise, but to allow prompt evolution of hydrogen gas from the reaction mixture. After addition is completed, the reaction is heated to 55°, with stirring for 1 hr. and then at 65° for 1.5 h, then allowed to cool. The cooled mixture is diluted with hexane, then 4 drops of acetic acid (gl.) are added, and the mixture poured into cold water (about 15°). The organic layer is recovered and washed thrice with portions of water, dried over anh. sodium sulfate, filtered and concentrated by evaporating under vacuum to obtain an oily residue. The residue is distilled in a Kugelrohr apparatus to obtain the refined product of this step, at 105—117° (air bath) and 0.133—0.127mBar (0.100—0.095 mm Hg).

*Step B* 2-cyanoethyl-decyl-dimethyl-silane (compound XIIa)

A mixture of 3.5 g of ethyl 4-sila-2-cyano-4,4-dimethyltetradecanoate (from Step A), 6 ml of acetic acid (gl.) and 4 ml. of concentrated hydrochloric acid is heated for 8 h in a bath at 120°, and by-products (e.g. ethyl acetate) are evaporated off. Vacuum is then applied and evaporation continued until a concentrate of mostly one phase remains. The concentrate is mixed with petroleum ether and water, and the organic phase recovered, washed with water, dried over anh. sodium sulfate, filtered and evaporated to obtain an oil. The oil is distilled in a Kugelröhr apparatus to obtain the title product of this step as fraction collected at 106—111° at 0.16—0.151 mBar (0.120—0.133 mm Hg), and then at 111—164° at 0.151—0.173 mBar (0.113—0.130 mm Hg). The fractions are combined for use in step C, below.

*Step C* 4-sila-4,4-dimethyltetradecanoic acid (compound IVc)

2.1 g of 2-cyanoethyl-n-decyl-dimethyl silane is refluxed with 2 g of potassium hydroxide and 8 g of water for 88 h with stirring. The mixture is cooled, acidified with 2N hydrochloric acid, then extracted with methylene chloride. The organic phase is dried over anh. sodium sulfate, filtered, and then evaporated under vacuum to obtain the title product as an oily residue, which is refined by distillation in a Kugelrohr apparatus at 112—116° at 0.14 to 0.147 mBar (0.110 to 0.105 mm Hg).

Example 6

N-[1'-phenyl-2'-p-tolylethyl]-3-(1-butyl-1-silacyclohexyl)-propanamide (compound No. 3)

*Step A* n-butyl-cyclopentamethylene-chlorosilane

In a separate vessel, under nitrogen, to a solution of 800 mg of cyclopentamethylenedichlorosilane in 10 ml of dry THF, is added a solution of 290 mg of n-butyl lithium in 2.9 ml of a dry hexane at a temperature of from about −10 to −16° with stirring. The mixture is continued stirring at room temperature for 30 min and held for use in step c.

*Step B* N-[1'-phenyl-2'-p-tolylethyl]-3-(1-butyl-1-silacyclohexyl)-1-propynamide (compound XVIb)

In a vessel, under an atmosphere of dry nitrogen at −30° a solution of 580 mg of n-butyl lithium in 5.8 ml of dry hexane is added to 900 mg of diisopropylamine in 10 ml of dry THF. The mixture stirred for 15 min., then cooled to −60°. A solution of 1.29 g of [(1'-phenyl-2'-p-tolyl)-ethyl] propiolamide (obtained according to Example 1A) in 10 ml of dry THF is added dropwise thereto while the temperature of the mixture is maintained between about −40 and −50°. The mixture is then stirred for 1 h at −25°. The solution of n-butyl-pentamethylene-chlorosilane prepared in step A is added dropwise, with stirring while maintaining the temperature at below about −10° and the mixture stirred for 3 h (at −20°).

Aqueous saturated ammonium chloride is added to the reaction mixture, and the organic phase recovered, dried over anh. sodium sulphate, and concentrated by evaporation *in vacuo* to obtain the crude product as an oil, which is refined by eluting through a silica gel column using chloroform as eluate to yield the product of this step as an oil, which crystallizes on standing to a solid (m.p. 87—90°).

*Step C* N-[1'-phenyl-2'-p-tolylethyl]-3-(1-butyl-1-silacyclohexyl)propanamide (compound No. 3)

To a solution of 1 g N-[1'-phenyl-2'-p-tolylethyl]-3-(1-butyl-1-silacyclohexyl)-1-propynamide in 100 ml of ethyl acetate in a hydrogenating apparatus, is added 300 mg of platinum oxide, and a pressure of 50 p.s.i. hydrogen is maintained for 24 hours with shaking. The reaction mixture is then filtered, and the filtrate concentrated (by evaporation *in vacuo*) to obtain the title product as an oil.

Example 7

N-[1'-phenyl-2'-p-tolylethyl]-3-(1-butyl-1-silacyclohexyl)-1-propenamide (compound XVb)

To a solution of 300 mg of N-[1'-phenyl-2'-p-tolylethyl]-3-(1-butyl-silacyclohexyl)-2-propynamide in 30 ml of ethanol in a hydrogenating apparatus, is added 50 mg of 5% palladium on calcium carbonate. The mixture is placed under 1 at. pressure of hydrogen and shaken until an equivalent of hydrogen gas had been taken up (about 45 min.). The reaction mixture is then filtered, and the filtrate concentrated (by evaporation *in vacuo*) to obtain the title product.

The following intermediate compounds can be prepared analogously to the foregoing examples and/or by the methods hereinbefore described.

TABLE I

("C" indicates the number of carbon atoms in the group linking Si to $\overset{O}{\overset{\|}{C}}$ or $-CH-OH$ and corresponding to A which is straight chain).

| Compound type/no. | | $R_1$ | $R_2$ | $R_3$ | R | C | |
|---|---|---|---|---|---|---|---|
| XVIc) | XVc) | $nC_3H_7$ | $CH_3$ | $CH_3$ | 1'-phenyl-2'-p-tolylethyl | 2 | |
| d) | d) | $nC_8H_{17}$ | $CH_3$ | $CH_3$ | | | |
| e) | e) | $t.C_4H_9$ | $CH_3$ | $CH_3$ | | | |
| f) | f) | $n.C_{10}H_{21}$ | $CH_3$ | $CH_3$ | | | |
| g) | g) | phenyl | $CH_3$ | $CH_3$ | | | oil |
| a) | h) | $CH_3$ | phenyl | phenyl | | | |
| i) | i) | benzyl | $CH_3$ | $CH_3$ | | | |
| j) | j) | $CH_3$ | benzyl | benzyl | | | |
| k) | k) | benzyl | $CH_3$ | phenyl | | | |
| l) | l) | p-tolyl | $CH_3$ | $CH_3$ | | | |
| m) | m) | $n.C_8H_{17}$ | phenyl | phenyl | | | |
| n) | n) | phenyl | phenyl | phenyl | | | |
| o) | o) | $n.C_4H_9$ | phenyl | phenyl | | | |
| b) | a) | $CH_3$ | $CH_3$ | $CH_3$ | | | |
| q) | q) | $CH_3$ | phenyl | phenyl | 1-indanyl | | |
| r) | r) | $CH_3$ | phenyl | phenyl | 1-ethoxycarbonyl-2-(3-indolyl)ethyl | | |
| s) | s) | $CH_3$ | phenyl | phenyl | 1'-methylbenzyl | | |
| a) | u) | $CH_3$ | phenyl | phenyl | 1'-phenyl-2'-p-tolylethyl | | |

TABLE I (Continued)

| Compound type/no. | $R_1$ | $R_2$ | $R_3$ | R | C | |
|---|---|---|---|---|---|---|
| II' (hal=Cl) | n.$C_{10}H_{21}$ | $CH_3$ | $CH_3$ | – | 4 | |
| IXb) | n.$C_{10}H_{21}$ | $CH_3$ | $CH_3$ | – | 2 | |
| IXc) | n.$C_{10}H_{21}$ | $CH_3$ | $CH_3$ | – | 5 | |
| IXd) | n.$C_{10}H_{21}$ | n.$C_6H_{13}$ | n.$C_6H_{13}$ | – | 3 | |
| IXe) | n.$C_8H_{17}$ | $CH_3$ | $CH_3$ | – | 5 | |
| IXf) | n.$C_4H_9$ | n.$C_3H_7$ | n.$C_3H_7$ | – | 9 | |
| IXg) | benzyl | $CH_3$ | $CH_3$ | – | 4 | |
| IVd) | n.$C_8H_{17}$ | $CH_3$ | $CH_3$ | – | 4 | |
| IVe) | benzyl | $CH_3$ | $CH_3$ | – | 4 | |
| IVf) | phenyl | $CH_3$ | $CH_3$ | – | 4 | |
| VI'b) | $CH_3$ | phenyl | phenyl | – | 3 | |
| VI'c) | n.$C_4H_9$ | phenyl | phenyl | – | 3 | |
| VI'd) | phenyl | phenyl | phenyl | – | 3 | |
| IVg) | $CH_3$ | phenyl | phenyl | – | 3 | |
| IVh) | n.$C_4H_9$ | phenyl | phenyl | – | 3 | |
| IVi) | phenyl | phenyl | phenyl | – | 3 | |
| IVj) | n.$C_{10}H_{21}$ | $CH_3$ | $CH_3$ | – | 5 | |
| IVk) | n.$C_{10}H_{21}$ | n.$C_6H_{13}$ | n.$C_6H_{13}$ | – | 3 | |
| IVl) | n.$C_8H_{17}$ | $CH_3$ | $CH_3$ | – | 5 | |
| IVm) | n.$C_4H_9$ | n.$C_3H_7$ | n.$C_3H_7$ | – | 9 | |
| IVn) | benzyl | $CH_3$ | $CH_3$ | – | 4 | |
| VI'e) | n.$C_{10}H_{21}$ | $CH_3$ | $CH_3$ | – | 4 | |
| VI'f) | n.$C_{10}H_{21}$ | $CH_3$ | $CH_3$ | – | 5 | |
| VI'g) | n.$C_{10}H_{21}$ | $CH_3$ | $CH_3$ | – | 7 | |
| IVo) | n-$C_{10}H_{21}$ | $CH_3$ | $CH_3$ | – | 7 | |

TABLE I (Continued)

| Compound type/no. XVI | Compound type/no. XV | $R_1$ | $R_2 + R_3$ | R | C | |
|---|---|---|---|---|---|---|
| u) | v) | $n.C_4H_9$ | $(CH_2)_5$ | 1-indanyl | 2 | |
| v) | w) | $n.C_4H_9$ | $(CH_2)_5$ | 1-ethoxycarbonyl-2-(3-indolyl)ethyl | 2 | |
| w) | x) | $n.C_4H_9$ | $(CH_2)_5$ | 1'-methylbenzyl | 2 | |
| y) | z) | $n.C_4H_9$ | $(CH_2)_5$ | 1'-benzyl-2'-phenyl-ethyl | 2 | |
| z) | aa) | $n.C_4H_9$ | $(CH_2)_4$ | 1'-phenyl-2'-p-tolylethyl | 2 | |
| aa) | bb) | $n.C_4H_9$ | $(CH_2)_6$ | 1'-phenyl-2'-p-tolylethyl | 2 | |
| bb) | cc) | $C_{10}H_{21}$ | $(CH_2)_5$ | 1'-phenyl-2'-p-tolylethyl | 2 | |
| cc) | dd) | $C_{10}H_{21}$ | $(CH_2)_4$ | 1'-phenyl-2'-p-tolylethyl | 2 | |
| dd) | ee) | $CH_3$ | $(CH_2)_5$ | 1'-phenyl-2'-p-tolylethyl | 2 | |
| ee) | ff) | $n.C_4H_9$ | $(CH_2)_{11}$ | 1'-phenyl-2'-p-tolylethyl | 2 | |
| ff) | gg) | benzyl | $(CH_2)_5$ | 1'-phenyl-2'-p-tolylethyl | 2 | |
| gg) | hh) | phenyl | $(CH_2)_5$ | 1'-phenyl-2'-p-tolylethyl | 2 | |
| hh) | ii) | $n.C_4H_9$ | $-(CH_2)_2-CH(CH_3)-(CH_2)_2-$ | 1'-phenyl-2'-p-tolylethyl | 2 | |

as well as
6,6,7,7-tetramethyl-6-sila-n-octanoic acid (IVp)
6,6-dibenzyl-6-sila-heptanoic acid ($IV_q'$);
N-benzyl-4,4-dimethyl-4-sila-2-pentynamide m.p. 73—76°,
N-benzyl-4,4-dimethyl-4-sila-2-pentenamide m.p. 68—72°,
as well as the compound XV and XVI corresponding to the compound Nos. 68 to 77.

In an analogous manner to the foregoing examples or according to any of the methods hereinbefore described, the following compounds of formula I can be obtained.

| Compound No. | $R_1$ | $R_2$ | $R_3$ | A | R | physical data |
|---|---|---|---|---|---|---|
| 4 | $CH_3$ | $CH_3$ | $CH_3$ | $(CH_2)_2$ | 1'-phenyl-2'-p-tolylethyl | m.p. 91–95° |
| 5 | $C_3H_7$ | $CH_3$ | $CH_3$ | | | oil |
| 6 | $n.C_8H_{17}$ | $CH_3$ | $CH_3$ | | | oil |
| 7 | $t.C_4H_9$ | $CH_3$ | $CH_3$ | | | oil |
| 8 | $n.C_{10}H_{21}$ | $CH_3$ | $CH_3$ | | | m.p. 39–42° |
| 9 | phenyl | $CH_3$ | $CH_3$ | | | gum |
| 10 | benzyl | $CH_3$ | $CH_3$ | | | oil |
| 11 | $CH_3$ | benzyl | benzyl | | | |
| 12 | benzyl | $CH_3$ | phenyl | | | |
| 13 | p-tolyl | $CH_3$ | $CH_3$ | | | |
| 14 | $n.C_8H_{17}$ | phenyl | phenyl | | | |
| 15 | phenyl | phenyl | phenyl | | | |
| 16 | $n.C_4H_9$ | phenyl | phenyl | | ↓ | m.p. 92–97° |
| 17 | $CH_3$ | $CH_3$ | $CH_3$ | | benzyl | oil |
| 18 | $CH_3$ | phenyl | phenyl | | 1-indanyl | |
| 19 | $CH_3$ | phenyl | phenyl | | 1-ethoxycarbonyl-2-(3-indolyl)ethyl | |
| 20 | $CH_3$ | phenyl | phenyl | ↓ | 1'-methylbenzyl | |

| Compound No. | $R_1$ | $R_2$ | $R_3$ | A | R | physical data |
|---|---|---|---|---|---|---|
| 22 | $C_{10}H_{21}$ | $CH_3$ | $CH_3$ | $(CH_2)_2$ | 1-indanyl | |
| 23 | $C_{10}H_{21}$ | $CH_3$ | $CH_3$ | ↓ | 1-ethoxycarbonyl-2-(3-indolyl)ethyl | m.p. 54° |
| 24 | $C_{10}H_{21}$ | $CH_3$ | $CH_3$ | | 1′-methylbenzyl | m.p. 34° |
| 25 | $C_{10}H_{21}$ | $CH_3$ | $CH_3$ | | o-methylphenyl | |
| 26 | $C_{10}H_{21}$ | $CH_3$ | $CH_3$ | | 1′-methylbenzyl | oil (+ isomer) |
| 27 | $C_{10}H_{21}$ | $CH_3$ | $CH_3$ | $(CH_2)_4$ | 1′-phenyl-2′-p-tolylether | m.p. 43–45° |
| 28 | $C_{10}H_{21}$ | $CH_3$ | $CH_3$ | $(CH_2)_3$ | 1′-benzyl-2′-phenylethyl | b.p. 197–214°/ 0.1 mm/Hg |
| 29 | $C_{10}H_{21}$ | $CH_3$ | $CH_3$ | $(CH_2)_3$ | 1-phenyl-2′-p-tolylethyl | b.p. 200–237°/ 0.1 mm/Hg |
| 30 | n.$C_8H_{17}$ | $CH_3$ | $CH_3$ | $(CH_2)_4$ | 1′-benzyl-2′-phenylethyl | |
| 31 | $(CH_3)_3$ | $CH_3C$ | $CH_3$ | $(CH_2)_4$ | ,, | |
| 32 | benzyl | $CH_3$ | $CH_3$ | $(CH_2)_4$ | ,, | |
| 33 | phenyl | $CH_3$ | $CH_3$ | $(CH_2)_4$ | ,, | |
| 34 | n.$C_8H_{17}$ | $CH_3$ | $CH_3$ | $(CH_2)_4$ | 1′-phenyl-2′-p-tolylethyl | |
| 35 | $(CH_3)_3$ | $CH_3C$ | $CH_3$ | $(CH_2)_4$ | 1′-phenyl-2′-p-tolylethyl | |
| 36 | benzyl | $CH_3$ | $CH_3$ | $(CH_2)_4$ | ,, | |
| 37 | phenyl | $CH_3$ | $CH_3$ | $(CH_2)_4$ | ,, | |
| 38 | $CH_3$ | phenyl | phenyl | $(CH_2)_3$ | ,, | |

| Compound No. | $R_1$ | $R_2$ | $R_3$ | A | R | physical data |
|---|---|---|---|---|---|---|
| 39 | $n.C_4H_9$ | phenyl | phenyl | $(CH_2)_3$ | 1'-phenyl-2'-p-tolylethyl | |
| 40 | phenyl | phenyl | phenyl | $(CH_2)_3$ | ,, | |
| 41 | $CH_3$ | benzyl | benzyl | $(CH_2)_4$ | ,, | |
| 42 | $n.C_{10}H_{21}$ | $CH_3$ | $CH_3$ | $(CH_2)_4$ | 1-indanyl | |
| 43 | $n.C_{10}H_{21}$ | $CH_3$ | $CH_3$ | $(CH_2)_4$ | 1-ethoxycarbonyl-2-(3-indolyl)ethyl | |
| 44 | $n.C_{10}H_{21}$ | $CH_3$ | $CH_3$ | $(CH_2)_4$ | 1'-methylbenzyl | |
| 46 | $n.C_{10}H_{21}$ | $CH_3$ | $CH_3$ | $(CH_2)_4$ | 1'-p-methylbenzyl-2'-p-tolylethyl | |
| 47 | $n.C_{10}H_{21}$ | $CH_3$ | $CH_3$ | $(CH_2)_4$ | 1'-methylbenzyl | + isomer |
| 48 | $n.C_{10}H_{21}$ | $CH_3$ | $CH_3$ | $(CH_2)_5$ | 1'-phenyl-2'-p-tolylethyl | |
| 49 | $n.C_{10}H_{21}$ | $n.C_6H_{13}$ | $n.C_6H_{13}$ | $(CH_2)_3$ | ,, | |
| 50 | $n.C_8H_{17}$ | $CH_3$ | $CH_3$ | $(CH_2)_5$ | ,, | |
| 51 | $n.C_4H_9$ | $n.C_3H_7$ | $n.C_3H_7$ | $(CH_2)_9$ | ,, | |
| 52 | $C_2H_5$ | $CH_3$ | $CH_3$ | $(CH_2)_7$ | ,, | |
| 68 | $n.C_{10}H_{21}$ | $CH_3$ | $CH_3$ | $(CH_2)_5$ | 1'-benzyl-2'-phenylethyl | |
| 69 | $n.C_{10}H_{21}$ | $n.C_6H_{13}$ | $n.C_6H_{13}$ | $(CH_2)_3$ | ,, | |
| 70 | $n.C_8H_{17}$ | $CH_3$ | $CH_3$ | $(CH_2)_5$ | ,, | |
| 71 | $n.C_4H_9$ | $n.C_3H_7$ | $n.C_3H_7$ | $(CH_2)_9$ | ,, | |

| Compound No. | $R_1$ | $R_2$ | $R_3$ | A | R | physical data |
|---|---|---|---|---|---|---|
| 72 | $C_2H_5$ | $CH_3$ | $CH_3$ | $(CH_2)_7$ | 1′-benzyl-2′-phenylethyl | |
| 73 | m-$CH_3O$ phenyl | $CH_3$ | $CH_3$ | $(CH_2)_2$ | ,, | m.p. 75° |
| 74 | n-$C_4H_9$ | $C_2H_5$ | $C_2H_5$ | $(CH_2)_2$ | 1′-phenyl-2′-p-tolylethyl | oil |
| 75 | n-$C_{10}H_{21}$ | $CH_3$ | $CH_3$ | $(CH_2)_2$ | 1′-benzyl-2′-phenylethyl | m.p. 70–71° |
| 76 | n-$C_{18}H_{37}$ | $CH_3$ | $CH_3$ | $(CH_2)_2$ | 1′-methylbenzyl | m.p. 61° |
| 77 | n.$C_{18}H_{37}$ | $CH_3$ | $CH_3$ | $(CH_2)_2$ | 1′-phenyl-2′-p-tolylethyl | m.p. 64° |
| 78 | n.$C_{10}H_{21}$ | $CH_3$ | $CH_3$ | $(CH_2)_2$ | 1′-phenyl-2′-p-tolylethyl | + isomer b.p. 200–235° (0.8 mm) |
| 79 | n.$C_{10}H_{21}$ | $CH_3$ | $CH_3$ | $(CH_2)_2$ | ,, | – isomer b.p. 195–227° (0.85 mm) |

| Compound No. | $R_1$ | $R_2 + R_3$ | A | R | physical data |
|---|---|---|---|---|---|
| 53 | $n.C_4H_9$ | $(CH_2)_5$ | $(CH_2)_2$ | 1-indanyl | |
| 54 | ↓ | $(CH_2)_5$ | ↓ | 1-ethoxycarbonyl-2-(3-indolyl)ethyl | |
| 56 | | $(CH_2)_5$ | | 1'-methylbenzyl | |
| 58 | | $(CH_2)_5$ | | 1'-benzyl-2'-phenylethyl | |
| 59 | | $(CH_2)_4$ | | 1'-phenyl-2'-p-tolylethyl | |
| 60 | | $(CH_2)_6$ | | ↓ | |
| 61 | $C_{10}H_{21}$ | $(CH_2)_5$ | | | |
| 62 | $C_{10}H_{21}$ | $(CH_2)_4$ | | | |
| 63 | $CH_3$ | $(CH_2)_5$ | | | |
| 64 | $n.C_4H_9$ | $(CH_2)_{11}$ | | | |
| 65 | benzyl | $(CH_2)_5$ | | | |
| 66 | phenyl | $(CH_2)_5$ | | | |
| 67 | $n.C_4H_9$ | $-(CH_2)_2-C(CH_3)-(CH_2)_2-$ | | | |

The following NMR data were obtained in $CDCl_3$. Figures are in ppm and digits in brackets indicate the number of protons.
s = singlets; d = doublets; m = multiplet; b = broad

Compound No. 1:
7.7—6.7 (m, 20), 5.2 (m, 1), 3.0 (m, 2), 2.25 (s, 3), 1.6—0.3 (m, 4), 0.65 (d, 3).

Compound No. 9:
7.6—6.8 (m, 14), 5.7 (m, 1), 5.2 (m, 1), 3.0 (d, 2), 2.3 (s, 3), 2.0 (m, 2), 1.0 (m, 2), 0.25 (s, 6).

Compound No. 10:
7.3—6.7 (m, 14), 5.9 (m, 1), 5.15 (m, 1), 2.95 (m, 2), 2.2 (s, 3), 2.0—0.5 (m, 4), 0.15 (s, 3), 0.05 (s, 2), −0.1 (s, 3).

Compound No. XVo):
7.7—6.7 (m, 19), 6.2 (m, 1), 5.2 (m, 1), 3.05 (d, 2), 2.25 (s, 3), 2.0 (d, 1), 1.7—0.5 (m, 10).

Compound No. XVIg):
7.4—6.8 (m, 14), 6.0 (m, 1), 5.2 (m, 1), 3.1 (d, 2), 2.25 (s, 3), 2.2 (s, 2), 0.2 (s, 6).

Compound No. 2:
7.3 (s, 10), 5.4 (d, 1), 4.6 (sextet, 1), 2.9 (d, 4), 1.0 (distorted t, 3), 0.5 (m, 4).

Compound No. 3:
7.25 (s, 5), 7.1 (m, 4), 5.85 (m, 1), 3.1 (d, 2), 2.25 (s, 3), 2.2—0.2 (m, 23).

Compound No. XVa):
7.3 (s, 5), 7.0 (s, 4), 6.35 (d, 1), 6.0 (m, 1), 5.35 (m, 1), 3.1 (d, 2), 2.3 :(s, 3), 1.25 (m, 1), 0.25 (d, 9).

**Claims**

1. Process for the production of a compound of formula I

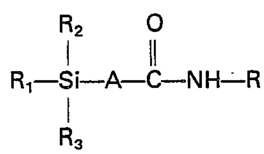

I

wherein R represents

a) an aralkyl radical of formula

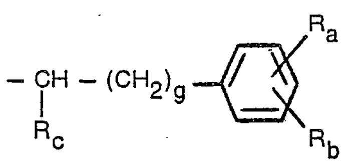

in which

g represents 0, 1 or 2,
$R_a$ represents hydrogen, fluorine, chlorine, bromine or iodine, $(C_{1-4})$alkyl or alkoxy or trifluoromethyl,
$R_b$ represents hydrogen, fluorine or chlorine, or $(C_{1-3})$alkyl or alkoxy,
$R_c$ represents i) hydrogen ii) a radical of formula

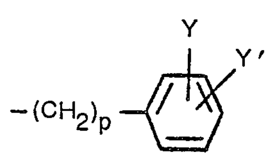

in which p represents 0, 1 or 2, and
Y represents hydrogen, fluorine, chlorine, bromine or iodine, or $(C_{1-4})$alkyl or alkoxy,
Y′ represents hydrogen, fluorine or chlorine or $(C_{1-3})$alkyl or alkoxy, or
iii) $(C_{1-8})$alkyl,

or R represents

b) an indolyl radical of formula

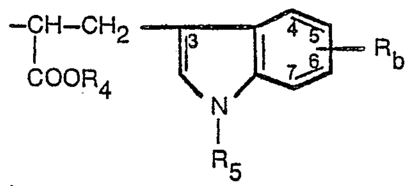

in which $R_b$ is as defined under a)

$R_4$ represents $(C_{1-8})$alkyl or unsubstituted benzyl, and

$R_5$ represents hydrogen, $(C_{1-8})$alkyl or unsubstituted benzyl,

or R represents

c) a benzocycloalkyl radical of formula

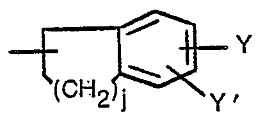

in which Y and Y′ are as defined under a) and

j represents 1, 2, 3, or 4,

$R_1$, $R_2$ and $R_3$ represent independently $C_{1-22}$alkyl or a radical of the formula

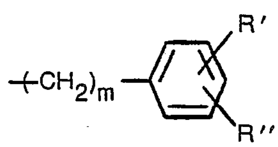

in which m represents 0, 1 or 2 and

R′ and R″ represent independently hydrogen, $C_{1-3}$alkyl or alkoxy, fluorine, chlorine, bromine or iodine

or

$R_1$ is as defined above and $R_2$ and $R_3$ together with the silicon atom to which they are attached form a 3 to 20 membered ring, and A is a $C_{2-20}$alkylene group, which comprises

a) reducing a compound of formula XV

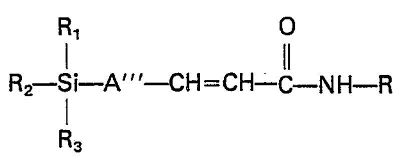

XV

or a compound of formula XVI

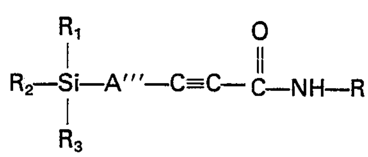

XVI

b) when A is $C_{3-20}$alkylene or A is ethylene and one of $R_1$, $R_2$ and $R_3$ is other than $C_{1-22}$alkyl, acylating a compound of formula III

$$H_2NR$$

with a compound of formula IV

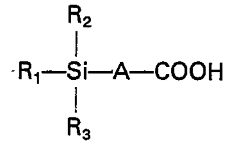 IV

or a reactive derivative thereof such as an active ester, carboimide adduct or a mixed anhydride of formula II

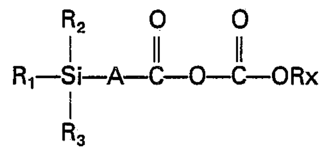

or an acid halide of formula II'

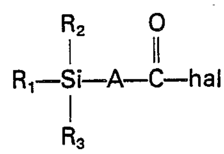 II'

whereby in the formulae XV, XVI, III, IV, II and II', $R_1$, $R_2$, $R_3$, R and A are as defined above, Rx represents $C_{1-4}$alkyl, A''' represents a group corresponding to A with two carbon atoms less and hal represents chlorine or bromine.

2. A compound of formula Ia

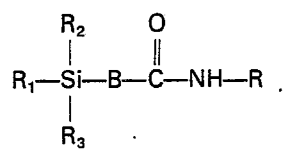 Ia

wherein R, $R_1$, $R_2$ and $R_3$ are as defined in Claim 1 and B represents a $C_{2-20}$alkylene radical whereby when B represents ethylene at least one of $R_1$, $R_2$ and $R_3$ is other than $C_{1-22}$alkyl.

3. A compound of formula XV as defined in claim 1.

4. A compound of formula XVI as defined in claim 1.

5. A pharmaceutical composition comprising a compound of formula Ia or XV as defined in claim 1 together with a pharmaceutically acceptable diluent or carrier.

6. A compound of formula Ia or XV as defined in claim 1 for use as a pharmaceutical.

7. A compound of formula Ia or XV as defined in claim 1 for use as a anti-artherosclerotic agent.

**Patentansprüche**

1. Verfahren zur Herstellung von Verbindungen der Formel I

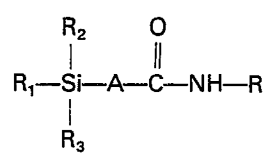 I

worin R

a) einen Aralkylrest der Formel

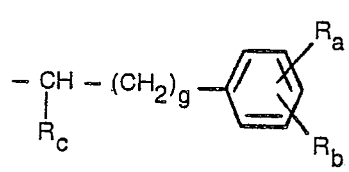

darstellt,

in welchem g 0, 1 oder 2 darstellt,

Ra Wasserstoff, Fluor, Chlor, Brom oder Iod, ($C_{1-4}$)-Alkyl oder Alkoxy oder Trifluormethyl-darstellt,
Rb Wasserstoff, Fluor oder Chlor, oder ($C_{1-3}$)-Alkyl oder Alkoxy darstellt,
Rc i) Wasserstoff
ii) einen Rest der Formel

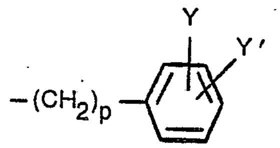

in welchem p 0, 1 oder 2 und
Y Wasserstoff, Fluor, Chlor, Brom oder Iod, oder ($C_{1-4}$)-Alkyl oder Alkoxy,
Y' Wasserstoff, Fluor oder Chlor oder ($C_{1-3}$)-Alkyl oder Alkoxy, darstellen oder
iii) ($C_{1-8}$)-Alkyl darstellt, oder R
b) einen Indolylrest der Formel

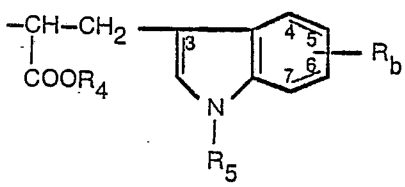

darstellt,
in welchem Rb wie unter a) definiert ist,
$R_4$ ($C_{1-8}$)-Alkyl oder unsubstituiertes Benzyl und
$R_5$ Wasserstoff, ($C_{1-8}$)-Alkyl oder unsubstituiertes Benzyl darstellen,
oder R
c) einen Bencycloalkylrest der Formel

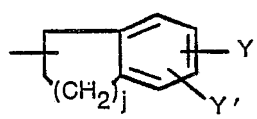

darstellt
in welchem Y und Y' wie unter a) definiert sind und j 1, 2, 3 oder 4 darstellt,
$R_1$, $R_2$ und $R_3$ unabhängig $C_{1-22}$—Alkyl oder einen Rest der Formel

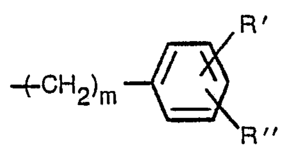

darstellen
in welchem m 0, 1 oder 2 und
R' und R'' unabhängig Wasserstoff $C_{1-3}$-Alkyl oder Alkoxy, Fluor, Chlor, Brom oder Iod darstellen oder $R_1$ wie oben definiert ist und $R_2$ und $R_3$ zusammen mit dem Siliconatom an das sie gebunden sind, einen 2-bis 20-gliedrigen Ring bilden, und A eine $C_{2-20}$-Alkylengruppe ist bestehend aus
a) Reduzierung einer Verbindung der Formel XV

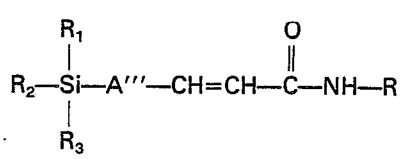

XV

oder einer Verbindung der Formel XVI

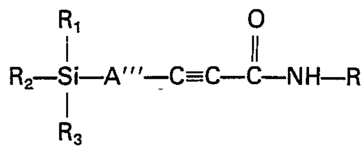

XVI

wenn A $C_{3-20}$-Alkylen oder A Ethylen ist und eines von $R_1$, $R_2$ und $R_3$ anders als $C_{1-22}$-Alkyl ist, Acylierung einer Verbindung der Formel III

$$H_2NR \qquad III$$

mit einer Verbindung der Formel IV

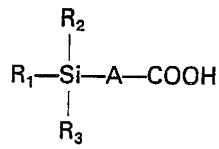

IV

oder einem reaktiven Derivat davon wie einen Aktivester, Carboimidaddukt oder einem gemischten Anhydrid der Formel II

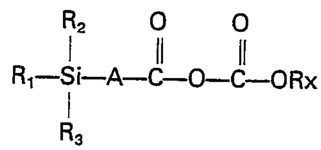

II

oder einem Säurehalid der Formel II′

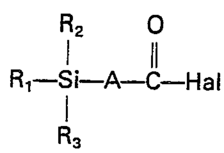

II′

wobei in der Formeln XV, XVI, III, IV, II und II′, $R_1$, $R_2$, $R_3$, R und A wie oben definiert sind, Rx $C_{1-4}$-Alkyl darstellt und A′′′ eine Gruppe entsprechend A mit zwei Kohlenstoffatomen weniger darstellt und Hal Chlor oder Brom darstellt.

2. Eine Verbindung der Formel Ia

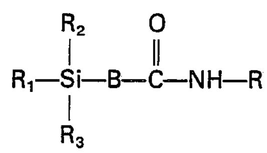

Ia

worin R, $R_1$, $R_2$ und $R_3$ wie im Anspruch 1 definiert sind und B einen $C_{2-20}$-Alkylenrest darstellt, wobei wenn B Ethylen darstellt, mindestens eines von $R_1$, $R_2$ und $R_3$ anders ist als $C_{1-22}$-Alkyl.

3. Eine Verbindung der Formel XV wie im Anspruch 1 definiert.

4. Eine Verbindung der Formel XVI wie im Anspruch 1 definiert.

5. Eine pharmazeutische Zusammensetzung enthaltend eine Verbindung der Formel Ia oder XV wie im Anspruch 1 definiert zusammen mit einem pharmazeutische annehmbaren Verdünner oder Träger.

6. Eine Verbindung der Formel Ia oder XV wie im Anspruch 1 definiert zur Verwendung als ein Pharmazeutikum.

7. Eine Verbindung der Formel Ia oder XV wie im Anspruch 1 definiert als ein anti-antherosclerotisches Mittel.

**Revendications**

1. Procédé de préparation d'un composé de formule I

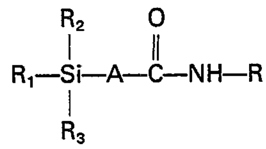

(I)

dans laquelle R représente

a) un radical aralkyle de formule

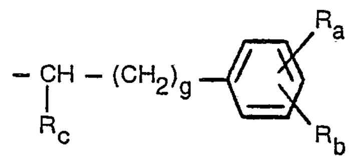

dans laquelle

g représente 0, 1 ou 2,

$R_a$ représente l'hydrogène, le fluor, le chlore, le brome ou l'iode, $(C_{1-4})$alkyle ou alcoxy ou trifluorométhyle,

$R_b$ représente l'hydrogène, le fluor ou le chlore ou $(C_{1-3})$alkyle ou alcoxy,

$R_c$ représente

i) l'hydrogène

ii) un radical de formule

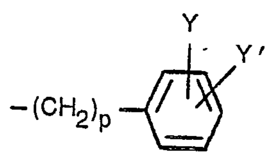

dans laquelle p représente 0, 1 ou 2, et

Y représente l'hydrogène, le fluor, le chlore, le brome ou l'iode, ou $(C_{1-4})$alkyle ou alcoxy,

Y' représente l'hydrogène, le fluor ou le chlore ou $(C_{1-3})$alkyle ou alcoxy, ou

iii) $(C_{1-8})$alkyle, ou bien R représente

b) un radical indolyle de formule

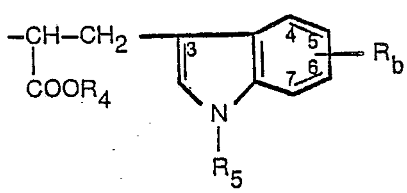

dans laquelle $R_b$ est comme défini sous a)

$R_4$ représente $(C_{1-8})$alkyle ou benzyle non substitué, et

$R_5$ représente l'hydrogène, $(C_{1-8})$alkyle ou benzyle non substitué,

ou bien R représente

c) un radical benzocycloalkyle de formule

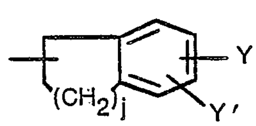

dans laquelle

Y et Y' sont comme définis sous a) et

j représente 1, 2, 3 ou 4,

$R_1$, $R_2$ et $R_3$ représentent indépendamment

$C_{1-22}$-alkyle ou un radical de formule

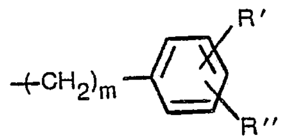

dans laquelle m représente 0, 1 ou 2 et

R′ et R″ représentent indépendamment l'hydrogène, $C_{1-3}$alkyle ou alcoxy, le fluor, le chlore, le brome ou l'iode ou bien

$R_1$ est comme défini ci-dessus et $R_2$ et $R_3$ ensemble avec l'atome de silicium auquel ils sont fixés forment un cycle ayant de 3 à 20 chaînons,

et A est un groupe $C_{2-20}$alkylène, qui comprend

a) la réduction d'un composé de formule XV

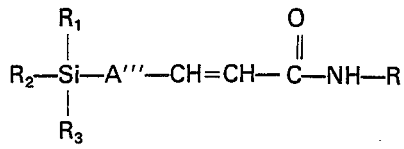 (XV)

ou d'un composé de formule XVI

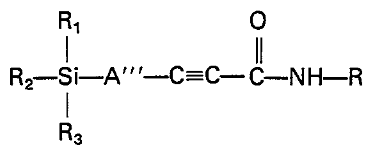 (XVI)

b) lorsque A est $C_{3-20}$alkylène ou A estéthylène et l'un de $R_1$, $R_2$ et $R_3$ est autre que $C_{1-22}$alkyle, l'acylation d'un composé de formule III

$$H_2NR \qquad \text{(III)}$$

avec un composé de formule IV

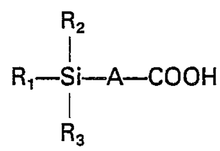 (IV)

ou un dérivé réactif de ce composé tel qu'un ester activé, un produit d'addition du carboimide ou un anhydride mixte de formule II

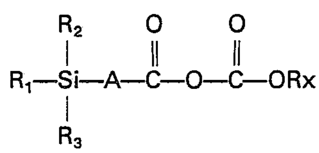 (II)

ou un halogénure d'acide de formule II′

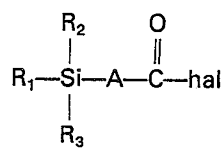 (II′)

$R_1$, $R_2$, $R_3$, R et A étant comme définis ci-dessus dans les formules XV, XVI, III, IV, II et II′, Rx représente $C_{1-4}$alkyle,

A‴ représente un groupe correspondant à A avec deux atomes de carbone en moins et Hal représente le chlore ou le brome.

2. Un composé de formule Ia

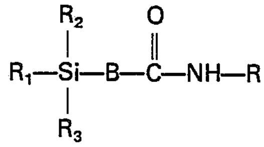

(Ia)

dans laquelle R, $R_1$, $R_2$ et $R_3$ sont comme définis à la revendication 1 et B représente un radical $C_{2-20}$alkylène, l'un au moins de $R_1$, $R_2$ et $R_3$ étant autre que $C_{1-22}$alkyle lorsque B signifie éthylène.

3. Un composé de formule XV comme défini à la revendication 1.

4. Un composé de formule XVI comme défini à la revendication 1.

5. Une composition pharmaceutique comprenant un composé de formule Ia ou XV comme défini à la revendication 1, ensemble avec un diluant ou véhicule pharmaceutiquement acceptables.

6. Un composé de formule Ia ou XV comme défini à la revendication 1 pour l'utilisation comme médicament.

7. Un composé de formule Ia ou XV comme défini à la revendication 1 pour l'utilisation comme agent anti-athéroscléreux.